# EUROPEAN PATENT APPLICATION

(11) **EP 1 935 444 A1**
(43) Date of publication of application: **25.06.2008**
(21) Application number: 06127186.2
(22) Date of filing: 22.12.2006
(51) Int. Cl.: A61M 5/50

(54) **A plunger for a dispensing member.**

(71) Applicant: Hubert De Backer NV, 9100 Sint Niklaas (BE)
(72) Inventor: De Backer, Jan Sr., 9100, SINT-NIKLAAS (BE)
(74) Representative: Quintelier, Claude

(57) **Abstract**

A plunger (5) for a dispensing system, said plunger (5) comprising a piston (7) and a drive rod (10) ending in a handhold (6), said drive rod (10) comprising a recess (9) applied in an outer wall of said drive rod (10), said recess (9) being dimensioned in such a manner as to cause a rupture of said drive rod (10) when pulling on said handhold (6).

## Description

The present invention relates to a plunger for a dispensing system, said plunger comprising a piston and a drive rod ending in a handhold.

Such a plunger and more in general, such a dispensing system is for example known from the international patent application W02005/016170. The plunger is housed inside the cartridge of the dispensing system and serves to push the product, stored in the cartridge, towards the cannula. For this purpose the drive rod is pushed through the cartridge by applying a pressure on the handhold.

A drawback of the known plungers is that they are often reused. After the dispensing system has been emptied for the first time, the user often removes the plunger from the cartridge and refills the latter with the product. In such a manner, the dispensing system can be refilled several times and reused. By acting however in this way, the probability that the dispensing system will be infected is rather high and this could lead to serious consequences, in particular when the injected product is a pharmaceutical product.

It is an object of the present invention to create a plunger for a dispensing system which can not be reused.

For this purpose, a plunger according to the present invention is characterised in that said drive rod comprises a recess applied in an outer wall of said drive rod, said recess being dimensioned in such a manner as to cause a rupture of said drive rod when pulling on said handhold. The presence of the recess causes the drive rod to be broken when there is pulled on the handhold. In such a manner, the plunger is broken and can not be reused, what means that it doesn't make sense to refill the cartridge as with a broken plunger it is no longer possible to push the product out of the cartridge. The presence of the recess doesn't affect the initial working of the dispensing system as the plunger will not break by applying a pressure.

A first preferred embodiment of a plunger according to the invention is characterised in that said drive rod comprises a locking ring, said recess being applied in a part of said plunger extending between a top of said piston and said locking ring. In such a manner, the recess is applied near the piston and a substantial part of the drive rod is pulled off when pulling on the handhold. The piston can thus no longer be operated and it becomes even difficult to remove the piston from the cartridge when the drive rod part has been pulled off.

A second preferred embodiment of a plunger according to the invention is characterised in that said recess extends circumferentially over said drive rod. The recess then extends over the circumference of the drive rod, thereby ensuring a reliable pull off.

The invention will now be described in more details with reference to the drawings.

In the drawings :
figure 1 illustrates an overall view of a dispensing system according to the present invention;
figure 2 illustrates a plunger according to the present invention; and
figure 3 illustrates the dispensing system of the invention with the pulled off plunger.

In the drawings a same reference sign has been allocated to a same or analogous element.

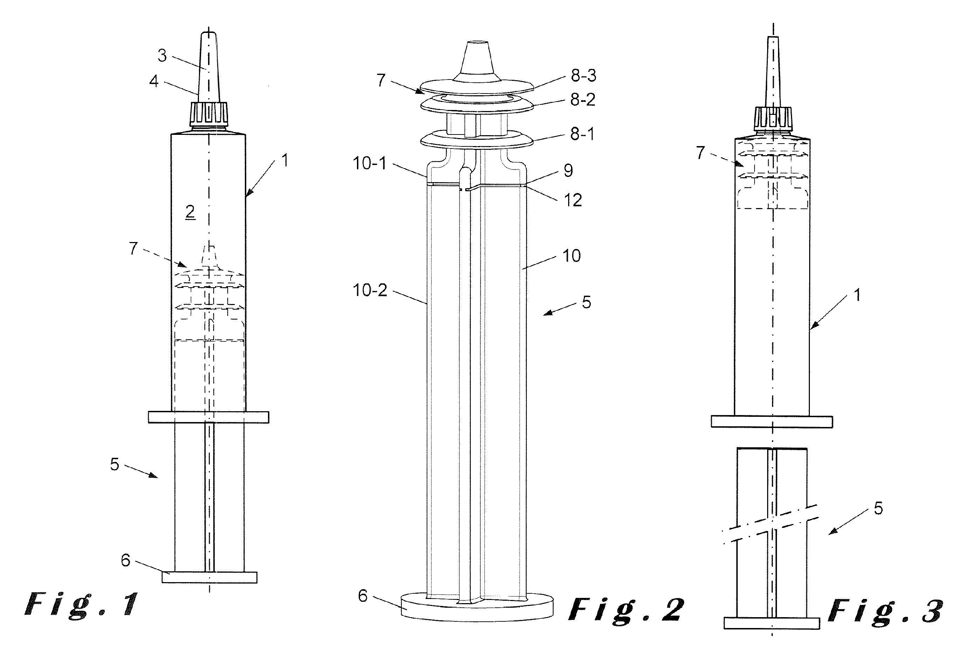

The dispensing system 1, illustrated in figure 1, comprises a cartridge 2 provided with a cannula 3 covered by a cap 4. A plunger 5 is movably mounted in the cartridge 2. The plunger is provided with a handhold 6. Upon use, the user pushes with his finger on the handhold 6, thereby causing the plunger to move inside the cartridge and push the product, contained into the cartridge, towards the cannula 3. The plunger is provided with a piston 7 forming the frontal part of the plunger. Preferably, the piston is formed by one or more discs 8-1, 8-2 and 8-3, which enable to scrape the inner wall of the cartridge, thereby taking care that no product remains on the inner wall of the cartridge.

As illustrated in figure 2, the plunger comprises a drive rod 10 extending between the piston 7 and the handhold 6. The drive rod comprises a recess 9 applied in an outer wall of the drive rod. Preferably, the recess extends circumferentially over the drive rod. The plunger is preferably formed by casting polyethylene. The recess is formed by providing in the mould a protuberance so that at the height of the recess less material is present and only a sheetlike connection is formed between the upper part 10-1 and the lower part 10-2 of the plunger.

The recess is dimensioned in such a manner as to cause a rupture of the drive rod when pulling on the handhold. Indeed, by only having a small amount of material connecting the upper and lower part of the plunger, this connection becomes weak. So, when the user pushes on the handhold, the recess is compressed and the inward movement of the plunger is not hindered. However, when the user would now like to pull the drive rod out of the cartridge, for example for refilling the cartridge, a pulling force is applied on the lower part 10-2 of the plunger, thereby causing the lower part to be torn off of the upper part. The upper part then remains inside the cartridge as illustrated in figure 3 and the lower part falls out of the cartridge thereby making the dispensing system no longer usable for refilling. In such a manner, there is avoided that the dispensing system is reused and the probability of using an infected dispensing system is substantially reduced.

The plunger is preferably provided with a locking ring 12 causing the plunger to be locked inside the cartridge when a pulling force is applied. In the latter case, the recess is applied in that part of the plunger extending between the locking ring and the top of the piston. In such a manner, the recess is close to the piston and it becomes impossible to remove the remaining part of the drive rod from the cartridge after pulling off the lower plunger part.

## Claims

1. A plunger for a dispensing system, said plunger comprising a piston and a drive rod ending in a handhold, **characterised in that** said drive rod comprises a recess applied in an outer wall of said drive rod, said recess being dimensioned in such a manner as to cause a rupture of said drive rod when pulling on said handhold.

2. A plunger as claimed in claim 1, **characterised in that** said drive rod comprises a locking ring, said recess being applied in a part of said plunger extending between a top of said piston and said locking ring.

3. A plunger as claimed in claim 1 or 2, **characterised in that** said recess extends circumferentially over said drive rod.

4. A plunger as claimed in any one of the claims 1 to 3, **characterised in that** at a location where said recess is applied, said drive rod is sheet-like shaped.
